# EUROPÄISCHE PATENTSCHRIFT

(11) **EP 1 553 830 B1**
(45) Veröffentlichungstag und Bekanntmachung des Hinweises auf die Patenterteilung: **23.02.2011**
(21) Anmeldenummer: 03748119.9
(22) Anmeldetag: 06.10.2003
(51) Int. Cl.: A01N 41/04, C02F 1/50, A01P 1/00

(54) **VERFAHREN ZUM ABTÖTEN VON MIKROORGANISMEN**
METHOD FOR THE DESTRUCTION OF MICROORGANISMS
PROCEDES POUR DETRUIRE DES MICRO-ORGANISMES

(30) Priorität: 07.10.2002 DE 10246625
(43) Veröffentlichungstag der Anmeldung: 20.07.2005
(73) Patentinhaber: BASF SE, 67056 Ludwigshafen (DE)
(72) Erfinder: DIETSCHE, Frank, 69198 Schriesheim (DE); BOUILLO, Nathalie, 76532 Baden-Baden (DE); KOLTER, Karl, 67117 Limburgerhof (DE); HAMERS, Christoph, 67071 Ludwigshafen (DE)
(86) Internationale Anmeldenummer: PCT/EP2003/011017
(87) Internationale Veröffentlichungsnummer: WO 2004/032628

(56) Entgegenhaltungen:
- EP-A- 0 130 789
- WO-A-02/49557
- WO-A-92/05695
- WO-A-02/072020
- GB-A- 2 072 508
- US-B1- 6 239 182
- PATENT ABSTRACTS OF JAPAN vol. 004, no. 019 (C-073), 16. Februar 1980 (1980-02-16) & JP 54 157822 A (MITSUBISHI CHEM IND LTD), 13. Dezember 1979 (1979-12-13) & JP 54 157822 A 13. Dezember 1979 (1979-12-13)

## Beschreibung

Die Erfindung betrifft ein Verfahren zum Abtöten von Mikroorganismen in wässrigen technischen Systemen oder Erzeugnissen für technische Anwendungen auf Wasserbasis, bei dem man dem System 0,001 bis 5 Gew.-% eines wasserlöslichen oder wasserdispergierbaren Polymers enthaltend 30 bis 98 mol % Styrolsulfonsäure, 2 bis 40 mol % eines N-Vinyllactams und/oder N-Vinylamins sowie 0 bis 30 mol % weiterer radikalisch polymerisierbarer Monomerer als biozides Additiv zugibt. Sie betrifft weiterhin ein Verfahren zum Schützen von Gegenständen durch Aufbringen einer antimikrobiell wirkenden wässrigen Zusammensetzung, die ein derartiges Additiv umfasst.

Der Begriff Mikrobiozid bzw. Biozid wird in einem allgemeinen Sinne für alle Substanzen verwendet, die in der Lage sind Mikroorganismen abzutöten und umfasst Antibiotika, Chemotherapeutika, Desinfektionsmittel oder Fungizide. In einem spezielleren Sinne umfasst der Begriff Substanzen, die in industriellen bzw. technischen Anwendungen eingesetzt werden, beispielsweise zur Konservierung von Kunststoffen, Farben, Papier, Holz oder zum Schutz industrieller Anlagen wie beispielsweise Kühlwasserkreisläufen. Nähere Einzelheiten sind in "Microbiocides", Ullmann's Encyclopedia of Industrial Chemistry, 6th Edition, 2000 Electronic Release dargestellt.

Bei technischen oder industriellen Anwendungen sind neben den üblichen Anforderungen an ein Biozid, wie beispielsweise gute biozide Wirkung aber keine Humantoxizität, auch noch besonders gute anwendungstechnische Eigenschaften erforderlich. In technischen Anwendungen verwendete Biozide sollen die Produkteigenschaften eines zu schützenden Produktes nicht beeinflussen und sollen ihre gute Wirkung im Produkt auch bei dessen weiterer Verarbeitung behalten. So soll z.B. beim Sprühtrocknen einer Acrylat-Dispersion, die ein Biozid enthält, die biozide Wirkung nicht beeinträchtigt werden, so dass auch noch das getrocknete Pulver oder das Redispergat durch das Biozid geschützt ist.

In wässrigen Dispersionen, beispielsweise zur Herstellung von Farben oder Beschichtungen, werden häufig definierte niedermolekulare Substanzen wie z.B. Bronopol (2-Brom-2-nitro-1,3 propandiol) als Biozid eingesetzt. Durch Bronopol kann jedoch ein leichter Gelbton verursacht werden. Bei manchen Anwendungen ist außerdem ein niedriger Gesamthalogengehalt zur Vermeidung von Korrosions- und Vergilbungsproblemen erwünscht. Weiterhin sind hochmolekulare Biozide in festen Filmen vorteilhaft, um eine hohe Langzeitstabilität zu gewährleisten.

Es ist bekannt, Polymere als Biozide einzusetzen. So offenbaren DE-A 199 40 023, DE-A 199 52 221 und DE-A 199 55 992 Copolymere von Aminopropylvinylethern, Acryloyloxyalkyldialkylaminen oder Acryloyloxyalkylbenzophenonammoniusalzen jeweils mit anderen ethylenisch ungesättigten Monomeren als mikrobieller Schutz für verschiedene industrielle Anwendungen oder zur Beschichtung von Oberflächen. Derartige kationische Polymere sind jedoch mit üblichen ionisch stabilisierten Acrylat-Dispersionen unverträglich.

Es ist weiterhin bekannt, Poly-4-styrolsulfonsäure oder die Salze davon für verschiedene Anwendungen im Bereich der Medizin einzusetzen. US 6,239,182 offenbart die Verwendung Na-Polystyrolsulfonaten als vaginales Empfängnisverhütungsmittel oder gegen HIV. US 6,290,946 offenbart die Verwendung Poly-4-styrolsulfonsäure oder Derivaten davon in Antibiotika.

Weiterhin ist die Verwendung von Verbindungen auf Basis von Styrolsulfon als antiadhäsive Verbindungen aus WO 02/072020 A2 bekannt.

Die Offenlegungsschrift GB 2 072 508 A offenbart antimykotische und antibakterielle Verbindungen, die Poly-p-Hydroxystyrole enthalten. In einer bevorzugten Ausführungsform kann es sich dabei um Sulfonsäurederivate handeln.

Aufgabe der Erfindung war es, ein Verfahren zum Abtöten von Mikroorganismen in wässriger Umgebung bereitzustellen, bei dem Polymere eingesetzt werden. Das Verfahren sollte weiterhin besonders bei ionisch stabilisierten Dispersionen zur Anwendung kommen ohne dass deren Eigenschaften dadurch negativ beeinflusst werden.

Dementsprechend wurden Verfahren zum Abtöten von Mikroorganismen in wässrigen technischen Systemen oder Produkten gefunden, bei dem man dem System ein biozides Additiv zusetzt, wobei es sich bei dem Biozid um 0,001 bis 5 Gew.-% eines wasserlöslichen oder wasserdispergierbaren Polymers enthaltend - jeweils bezogen auf die Gesamtmenge aller im Polymer vorhandenen Monomereinheiten-
(a) 30 bis 98 mol % Styrolsulfonsäure,
(b) 2 bis 40 mol % eines N-vinyllactams und/oder N-Vinylamins, sowie
(c) 0 bis 30 mol % weiterer radikalisch polymerisierbarer Monomerer
   handelt und die Summe aus (a), (b) und (c) 100 mol % ergibt.

In einer zweiten Ausführungsform der Erfindung wurde ein Verfahren zum Schützen von Gegenständen gefunden, bei dem man eine antimikrobiell wirkende Zusammensetzung, mindestens umfassend Wasser oder ein überwiegend wasserhaltiges Lösemittelgemisch sowie ein biozides Additiv mittels einer geeigneten Methode auf den Gegenstand aufbringt und Wasser oder das überwiegend wasserhaltige Lösemittelgemisch entfernt, wobei es sich bei dem Biozid um 0,001 bis 5 Gew.-% mindestens eines wasserlöslichen oder wasserdispergierbaren Polymers enthaltend - jeweils bezogen auf die Gesamtmenge aller im Polymer vorhandenen Monomereinheiten-
(a) 30 bis 98 mol % Styrolsulfonsäure,
(b) 2 bis 40 mol % eines N-Vinyllactams und/oder N-Vinylamins, sowie
(c) 0 bis 30 mol % weiterer radikalisch polymerisierbarer Monomerer
   handelt und die Summe aus (a), (b) und (c) 100 mol % ergibt.

Zu der Erfindung ist im Einzelnen das Folgende auszuführen.

Die Erfindung betrifft die Anwendung bestimmter biozider Additive im nichtmedizinischen Bereich. Therapeutische oder medizinische Anwendungen im oder am menschlichen oder tierischen Körper sowie Anwendungen im Pflanzenschutz sind von der vorliegenden Erfindung nicht umfasst.

Unter dem Begriff "wässrige technische Systeme" sollen im Rahmen dieser Erfindung Anlagen, insbesondere chemische Anlagen, Produktiorisanlagen oder Maschinen verstanden werden, in denen Wasser oder überwiegend wässrige Gemische als Hilfsstoffe oder Reaktionsmedien eingesetzt werden. Beispiele umfassen Reaktionskessel, Vorratskessel, Heizkessel, Wasser-Kühlkreisläufe, Wärmetauscherkreisläufe, Brauchwasserkreisläufe, Ballastwassertanks oder Klimaanlagen.

Unter dem Begriff "Erzeugnisse für technische Anwendungen auf Wasserbasis" sollen im Rahmen dieser Erfindung Erzeugnisse auf Wasserbasis verstanden werden, die im technischen, industriellen, gewerblichen, handwerklichen oder im Bereich des Haushaltes eingesetzt werden. Der Begriff umfasst auch den Bereich der Lebensmittelindustrie.

Der Begriff "Wasserbasis" bedeutet in prinzipiell bekannter Art und Weise, dass das in den Erzeugnissen verwendete Löse- oder Verdünnungsmittel zum überwiegenden Teil aus Wasser besteht und nur geringe Mengen von mit Wasser mischbaren oder darin dispergierbaren organischen Lösemitteln zusätzlich vorhanden sind. Bevorzugt besteht das Lösemittel nur aus Wasser.

Beispiele für Erzeugnisse auf Wasserbasis umfassen insbesondere Beschichtungsstoffe, Anstrichstoffe, Tränkstoffe, Wasserbasislacke oder Farben, Druckfarben, wie beispielsweise Flexodruckfarben oder Ink-Jet-Tinten, Dispersionen wie beispielsweise Acrylat-, Styrol-Acrylat-Dispersionen, sowie die Formulierungen derartiger Dispersionen zur Anwendung beispielsweise als Wandfarbe, Lackierung, Textilhilfsmittel. Weitere Beispiele umfassen PolyurethanDispersionen und deren Verwendung, beispielsweise zur Herstellung von Klarlacken für Holz, Papier oder Kunststofflackierungen.

Bei dem im erfindungsgemäßen Verfahren eingesetzten polymeren bioziden Additiv handelt es sich um ein wasserlösliches oder zumindest wasserdispergierbares Polymer. Bevorzugt ist das Polymer wasserlöslich.

Als Monomer (a) enthält das erfindungsgemäß eingesetzte Polymere 30 bis 98 mol % Styrolsulfonsäure bezüglich der Summe aller Monomerbausteine. Bevorzugt handelt es sich beim dem Monomer um 4-Styrolsulfonsäure, es kann sich jedoch auch um 2- oder 3-Styrolsulfonsäure oder um Gemische der 3 Isomeren handeln. Das erfindungsgemäß eingesetzte Polymer enthält bevorzugt 50 bis 90 mol % Styrolsulfonsäure und besonders bevorzugt 60 bis 80 mol %.

Als Comonomer (b) werden 2 bis 40 mol % eines N-Vinyllactams und/oder N-Vinylamins eingesetzt. Beispielsweise können N-Vinylpyrrolidon, N-Vinylpiperidon, N-Vinylcaprolactam oder N-Vinylformamid eingesetzt werden. Es können auch Gemische verschiedener N-Vinyllactame und/oder verschiedener N-Vinylamine eingesetzt werden. Bevorzugte sind N-vinylpyrrolidon und N-Vinylcaprolactam, besonders bevorzugt ist N-vinylpyrrolidon. Das erfindungsgemäß eingesetzte Polymer enthält bevorzugt 3 bis 30 mol % des Comonomers (b) und besonders bevorzugt 5 bis 20 mol %.

Optional können weitere Comonomere (c) mit olefinisch ungesättigten Gruppen eingesetzt werden, die radikalisch polymerisierbar sind. Derartige Monomere dienen zur Feineinstellung der Eigenschaften des Polymers. Art und Menge eines weiteren Monomers werden vom Fachmann je nach den gewünschten Eigenschaften des Polymers ausgewählt. Es können prinzipiell alle radikalisch polymerisierbaren Monomere eingesetzt werden, vorausgesetzt, es werden keine unerwünschten Eigenschaften erhalten. Insbesondere muss das Polymer wasserlöslich oder wasserdispergierbar bleiben. Bevorzugt handelt es sich bei Monomer (c) um Derivate ungesättigter Carbonsäuren, wie (Meth)Acrylester, Acrylamide oder Acrylnitril. Beispiele umfassen (Meth)acrylsäuremethylester, (Meth)acrylsäureethylester, (Meth) acrylsäure-n-propylester, (Meth)acrylsäureisopropylester, (Meth)acrylsäurelaurylester, (Meth)acrylsäurestearylester und die Ester der (Meth)acrylsäure die sich von den isomeren Butanolen ableiten, sowie Hydroxyethyl(meth)acrylat, Hydroxymethyl(meth)acrylat, Hydroxypropyl(meth)acrylat, Hydroxybutylacrylat oder Hydroxybutyl (meth) acrylat. Geeignete Monomere umfassen weiterhin ungesättigte Alkohole und Amine und Derivaten wie beispielweise Vinylalkohol, Vinylacetat, Vinylpropionat, Vinylsterat, Vinylbenzoat, Vinylmaleat, Vinylbutyral, Allylphthalat oder Allylmelamin. Weiterhin können auch ethylenisch ungesättigte Kohlenwasserstoffe, wie beipsielsweise Ethylen, Propylen oder Styrol eingesetzt werden. Es können selbstverständlich auch Gemische verschiedener Comonomerer (c) eingesetzt werden.

In einer besonderen Ausführungsform kann es sich bei den Comonomeren (c) auch um solche handeln, die neben einer ethylenisch ungesättigten Gruppe zusätzlich vernetzbare Gruppen aufweisen. Bei Verwendung derartiger Comonomerer werden Polymere erhalten, die zusätzliche Funktionalitäten aufweisen, die zur Vernetzung verwendet werden können. Beispielweise kann es sich dabei um Acetoacetoxyethyl-Methacrylat handeln.

Das erfindungsgemäß eingesetzte Polymer enthält üblicherweise 0 bis 30 mol % des Comonomers (c), bevorzugt 1 bis 20 mol % und besonders bevorzugt 5 bis 15 mol %.

Die Summe der im Polymer enthaltenen Monomere (a), (b) und (c) beträgt 100 mol %.

Die radikalische Polymerisation der Monomere bietet keinerlei Besonderheiten und kann nach dem Fachmann prinzipiell bekannten Methoden durchgeführt werden, z. B. als Emulsions-, Dispersions-oder Lösungspolymerisation oder inverser Suspensionspolymerisation durch Polymerisation in Substanz. Bevorzugt ist die Polymerisation in Lösung, wie beispielsweise von EP-A 130 789 beschrieben.

Das Gewichtsmittel des Molekulargewichtes beträgt üblicherweise 10.000 bis 500.000 g/mol, auch wenn in Spezialfällen auch außerhalb dieses Bereiches gute Ergebnisse erzielt werden können. Bevorzugt beträgt das Gewichtsmittel 15.000 bis 300.000 g/mol und besonders bevorzugt 20.000 bis 200.000 g/mol. Die Polydispersitäten M_{w}/Mₙ liegen üblicherweise zwischen 1,3 und 10 und sind vorzugsweise < 5 und besonders bevorzugt < 3. Es können aber auch außerhalb dieser Bereiche noch akzeptable Ergebnisse erzielt werden.

Die für das erfindungsgemäße Verfahren verwendeten Polymere sind wasserlöslich oder wasserdispergierbar. Die Sulfonsäuregruppen der Styrolsulfonsäureeinheiten des Polymers können als freie Säuregruppen vorliegen. Sie können aber auch ganz oder teilweise in Salze umgewandelt werden. Bevorzugt sind Alkalimetallsalze und besonders bevorzugt Na-Salze. Dies gilt auch für eventuell im Polymer vorhandene Comonomere (c), welche Säuregruppen aufweisen.

Das biozide Additiv wird wässrigen industriellen Systemen, also beispielsweise dem Kühl- oder Wärmetauscherkreislauf, oder aber dem Erzeugnis, also beispielsweise der wässrigen Dispersion, zugesetzt. Es kann das Additiv als solches zugegeben werden. Bevorzugt werden aber die besagten Konzentrate zugegeben. Der Fachmann wird weiterhin auf eine möglichst gleichmäßige Verteilung im zu schützenden Produkt bzw. System achten. Die verwendete Konzentration richtet sich nach dem gewünschten Einsatzzweck und wird vom Fachmann entsprechend gewählt. So wird der Fachmann zur langfristigen Prävention im Regelfalle nur eine relativ geringe Menge des bioziden Additivs einsetzen. Muss ein plötzlicher Befall mit Mikroorganismen bekämpft werden, so wird er eine höhere Konzentration auswählen.

Das biozide Additiv wird üblicherweise in Mengen von 0,001 bis 5 Gew.-% eingesetzt. Bevorzugt sind 0,005 bis 1 Gew.-% und besonders bevorzugt sind 0,01 bis 0,5 Gew.-%.

Diese Konzentrationen beziehen sich auf die Anwendung des bioziden Additivs. Es können selbstverständlich auch Konzentrate des Polymers hergestellt werden, die erst zur Anwendung auf die gewünschte Konzentration verdünnt werden.

Die erfindungsgemäß verwendeten wasserlöslichen oder wasserdispergierbaren Polymere können als einziges biozides Additiv eingesetzt werden. Sie wirken sowohl gegen Bakterien wie gegen Pilze und Algen. Selbstverständlich können auch Mischungen verschiedener wasserlöslicher oder wasserdispergierbarer Polymerer gemäß obiger Definition eingesetzt werden. Es können auch noch andere biozide Additive oder andere Hilfsstoffe eingesetzt werden, vorausgesetzt die es treten keine unerwünschten Effekte auf.

Die erfindungsgemäß eingesetzten Polymere eignen sich besonders zum Schutz von wässrigen Dispersionen sowie Produkten, in denen die wässrigen Dispersionen eingesetzt werden. Beispiele geeigneter Dispersionen umfassen insbesondere Dispersionen auf Basis von Acrylaten, wie Styrol-Acrylat-Dispersionen aber auch von Butadien-Styrol-Dispersionen oder Polyurethandispersionen. Sie können derartigen Dispersionen zugesetzt werden, ohne negative Effekte hervorzurufen. Beispielsweise können die erfindungsgemäß geschützten Dispersionen sprühgetrocknet werden, ohne dass die Wirkung des Biozides dabei wesentlich vermindert wird.

Das oben erwähnte Polymer kann auch in einem Verfahren zum Schützen von Gegenständen durch Aufbringen einer antimikrobiell wirkenden wässrigen Zusammensetzung auf die Oberfläche des Gegenstandes eingesetzt werden.

Die wässrige Zusammensetzung zum Beschichten umfasst ein Wasser oder überwiegend wasserhaltiges Lösemittelgemisch, das oben geschilderte biozide Additiv sowie optional eines oder mehrere Bindemittel sowie weitere Hilfsstoffe und Additive.

"Überwiegend wasserhaltig" bedeutet, dass der größere Teil des Lösemittelgemisches aus Wasser besteht und nur kleinere Mengen von nicht mehr als 25 %, bevorzugt nicht mehr als 15 und ganz besonders bevorzugt nicht mehr als 5 Gew.-% bezüglich der Menge aller Lösemittel eines oder mehrerer weiterer Co-Lösemittel vorhanden sind. Geeignete Co-Lösemittel sollten mit Wasser mischbar sein, insbesondere kann es sich dabei um Alkohole handeln.

Das biozide Additiv ist üblicherweise in Mengen von 0,001 bis 5 Gew.-% vorhanden. Bevorzugt sind 0,005 bis 1 Gew.-% und besonders bevorzugt sind 0,01 bis 0,5 Gew.-%.

Bevorzugt enthält die Zusammensetzung ein Bindemittel. Bei dem Bindemittel kann es sich um ein wasserlösliches Bindemittel handeln. Als Beispiel sei Polyvinylalkohol genannt. Bevorzugt handelt es sich bei dem Bindemittel aber um ein in Wasser zwar nicht lösliches, aber darin dispergierbares Bindemittel, z.B. um eine Acrylat-Dispersion.

Die Zusammensetzung kann weiterhin geeignete Additive und Hilfsmittel, wie beispielsweise Dispergierhilfsmittel, weitere Biozide, Haftvermittler oder Farbstoffe enthalten. Sie wird durch intensives Mischen aller Komponenten der Zusammensetzung mit dem Lösemittel hergestellt.

Insbesondere kann die Zusammensetzung auch noch einen Vernetzer oder ein Vernetzersystem enthalten. Die Art des Vernetzers richtet sich nach der vorgesehenen Anwendung der Zusammensetzung. Beispielsweise kann es sich um einen Vernetzer handeln, der beim Kontakt mit Luftsauerstoff die Vernetzung auslöst. Es kann sich auch um ein Vernetzersystem handeln, welches thermisch oder fotochemisch gehärtet werden kann. Die Komponenten des Vernetzers können in der Zusammensetzung von Anfang an enthalten sein. Es ist aber auch möglich, vernetzende Komponenten erst kurz vor der Anwendung zuzugeben.

Die Zusammensetzung wird auf den zu schützenden Gegenstand aufgebracht, beispielsweise durch Beschichten, Besprühen oder Eintauchen. Anschließend wird das Lösemittel entfernt, in der Regel durch einfaches Abdampfen des Lösemittels. Falls Vernetzung gewünscht ist, kann die Vernetzung durch Bestrahlung oder Erwärmung ausgelöst werden, oder sie erfolgt bereits beim Ausbringen auf den zu schützenden Gegenstand.

Die Zusammensetzung kann sowohl zur Beschichtung für den Innenals auch für Außenbereich eingesetzt werden. Das Additiv verändert nicht die primären Endeigenschaften der Beschichtung, sondern macht die Beschichtung nur langzeitstabiler gegen Verkeimung, Verpilzung und/oder Veralgung.

Die folgenden Beispiele sollen die Erfindung näher erläutern:

### Beispiel 1:

### Test in einer Styrol / Acrylat - Dispersion

Prüfvorschrift:
Je 20 ml der Probe wurde mit 0,2 ml der einzelnen Keimsuspensionen beimpft, homogenisiert und bei 25°C inkubiert. Die Keimzahl wurde sofort sowie nach 14 und 28 Tagen bestimmt. Dazu wurde die belastete Probe in entsprechenden Verdünnungen auf CASO-Agar mit Enthemmungsmittel ausgespatelt oder mit dem Agar als Gußplatte vermischt. Die Verdünnung der belasteten Probe erfolgte durch Homogenisierung mit Caso-Bouillon + Nr. 3 und einer Inkubation für 30 min im Wasserbad bei 40°C. Zum Nachweis der Bakterien wurden CASO-Agarplatten 3-5 Tage bei 30-35 °C bebrütet.

Durchführung der Tests:
Für die Tests wurde als biozides Additiv Na-Polystyrolsulfonat mit einem M_{w} von 200.000 g/mol eingesetzt.

Eine handelsübliche, wässrige Styrol/n-Butylacrylat Polymerdispersion mit einem Feststoffgehalt von 50 %, einer Teilchengröße von 170 nm und einer Viskosität von 400 mPas (Acronal S 728, Fa. BASF) wurde jeweils mit 500 , 1000 und 2000 ppm des bioziden Additivs dotiert. Die Eigenschaften der Dispersion veränderten sich durch den Zusatz des Biozids nicht. Es wurde ein Keimbelastungstest gemäß der o.g. Prüfungsbeschreibung mit *Escherichia coli, Pseudomonas aeruginosa, Staphylococcus aureus* durchgeführt. Die Ergebnisse sind in Tabelle 1 aufgeführt.

**Tabelle 1: Ergebnisse der Tests von Beispiel 1**

| Probe | Konzentration-des bioziden Additivs [ppm] | Zeit | Escherichia Coli | Pseudo-monas aeruginosa | Styphylococcus aureus |
|---|---|---|---|---|---|
| 0 | ohne Biozid | 48 h | 10⁴ | 10⁵ | 10⁵ |
| 1 | 500 | 24 h | 6000 | 0 | 12000 |
| 2 | 500 | 48 h | 0 | 0 | 0 |
| 3 | 1000 | 24 h | 8000 | 0 | 12000 |
| 4 | 1000 | 48 h | 0 | 0 | 0 |
| 5 | 2000 | 24 h | 3600 | 100 | 8000 |
| 6 | 2000 | 48 h | 0 | 0 | 0 |

### Beispiel 2:

Für die Tests wurde als biozides Additiv Na-Polystyrolsulfonat/ N-Vinylpyrrolidon (90/10) mit einem M_{w} von 30.000 g/mol eingesetzt.

Eine keimfreie wässrige Lösung wurde jeweils mit 10, 100 und 1000 ppm des bioziden Additivs dotiert. Es wurde ein Keimbelastungstest gemäß der Prüfung nach Ph. Eur. 3, 2000 (5.1.3) mit *Escherichia coli*, *Pseudomonas aeruginosa, Staphylococcus aureus* durchgeführt. Die Ergebnisse sind in Tabelle 2 aufgeführt. Die Proben wurden nach 3 Monaten bei Raumtemperatur noch einmal vermessen wobei keine Erhöhung der Keime festgestellt werden konnte.

**Tabelle 2 Ergebnisse der Tests von Beispiel 2**

| Nr. | c [ppm] | pH | Escherichia coli | nach 14 Tagen | Pseudomonas aeruginosa | nach 14 Tagen | Staphyl ococcus aureus | nach 14 Tagen |
|---|---|---|---|---|---|---|---|---|
| 1 | 10 | 7 | 4,7*10⁵ | <10 | 4,7*10⁵ | <10 | 4,7*10⁵ | <100 |
| 2 | 100 | 7 | 2,3*10⁵ | <10 | 2,3*10⁵ | <10 | 2,3*10⁵ | <10 |
| 3 | 1000 | 7 | 1,4*10⁵ | <10 | 1,4*10⁵ | <10 | 1,4*10⁵ | <10 |

### Beispiel 3:

### Sprühtrocknen einer Dispersion

Für die Trocknung wurde als biozides Additiv Na-Polystyrolsulfonat/N-Vinylpyrrolidon (95/5) mit einem M_{w} von 20.000 g/mol in einer 20%igen wässrigen Lösung eines Vinylpyrrolidon/Vinylacetat-Copolymeren (Monomerenverhältnis ca. 55/65; M_{w} 45000 - 70000 g/ mol) (Kollidon® VA 64, BASF AG) eingesetzt.

Die Lösung wurde in einem Eindüsensprühtrockner bei Eingangstemperaturen von über 140°C gefahren. Die Konzentration des polymeren Biozids konnte im Rahmen der Bestimmungsfehler wiedergefunden werden. Eine wiederholte Impfung wie in Beispiel 1 beschrieben zeigte eine unveränderte biozide Wirkung.

**Tabelle 3: Ergebnisse von Beispiel 3 (Angaben in Gew.-%)**

| | | | |
|---|---|---|---|
| Konzentration des Biozids [%] | 0,01 | 0,1 | 1 |
| Restfeuchte [%] | 2,0 | 2,1 | 2,4 |
| Konzentration im Pulver [%] | 0,05 | 0,49 | 5,05 |
| Farbe | farblos | farblos | farblos |
| pH-Wert | 6,1 | 6,2 | 6,3 |
| Feststoffgehalt [%] | 98 | 97,9 | 97,6 |

### Beispiel 4:

### Test in Milch

Eine Milchprobe wurde mit 0,01 Gew.-% des in Beispiel 1 beschriebenen Na-Polystyrolsulfonates versetzt. Die Probe war nach 5 Tagen geschlossener Lagerung bei Raumtemperatur noch stabil (Keimzahl, Aussehen, Geruch).

Eine Vergleichsprobe war schon nach 24 h geschlossener Lagerung bei Raumtemperatur deutlich verkeimt.

## Patentansprüche

1. Verfahren zum Abtöten von Mikroorganismen in wässrigen technischen Systemen oder Erzeugnissen für technische Anwendungen auf Wasserbasis, bei dem man dem System oder dem Erzeugnis ein biozides Additiv zusetzt, **dadurch gekennzeichnet, dass** es sich bei dem Biozid um 0,001 bis 5 Gew.-% mindestens eines wasserlöslichen oder wasserdispergierbaren Polymers enthaltend - jeweils bezogen auf die Gesamtmenge aller im Polymer vorhandenen Monomereinheiten-
(a) 30 bis 98 mol % Styrolsulfonsäure,
(b) 2 bis 40 mol % eines N-Vinyllactams und/oder N-Vinylamins, sowie
(c) 0 bis 30 mol % weiterer radikalisch polymerisierbarer Monomerer
handelt, und die Summe aus (a), (b) und (c) 100 mol % ergibt.

2. Verfahren gemäß Anspruch 1, **dadurch gekennzeichnet, dass** die Sulfonsäuregruppen ganz oder teilweise als Salz vorliegen.

3. Verfahren gemäß Anspruch 1 oder 2, **dadurch gekennzeichnet, dass** es bei den technischen Erzeugnissen auf Wasserbasis um wässrige Dispersionen handelt.

4. Verfahren gemäß Anspruch 3, **dadurch gekennzeichnet, dass** die Dispersion elektrostatisch bzw. ionisch stabilisiert ist wird.

5. Verfahren gemäß Anspruch 3, **dadurch gekennzeichnet, dass** die Dispersion sprühgetrocknet wird.

6. Verfahren gemäß Anspruch 1 oder 2, **dadurch gekennzeichnet, dass** es sich bei den wässrigen technischen Systemen um Kühl- oder Wärmetauscherkreisläufe handelt.

7. Verfahren zum Schützen von Gegenständen, bei dem man eine antimikrobiell wirkende Zusammensetzung, mindestens umfassend Wasser oder ein überwiegend wasserhaltiges Lösemittelgemisch sowie ein biozides Additiv mittels einer geeigneten Methode auf den Gegenstand aufbringt und Wasser oder das überwiegend wasserhaltige Lösemittelgemisch entfernt, **dadurch gekennzeichnet, dass** es sich bei dem Biozid um 0,001 bis 5 Gew.-% mindestens eines wasserlöslichen oder wasserdispergierbaren Polymers enthaltend - jeweils bezogen auf die Gesamtmenge aller im Polymer vorhandenen Monomereinheiten-
(a) 30 bis 98 mol % Styrolsulfonsäure,
(b) 2 bis 40 mol % eines N-Vinyllactams und/oder N-Vinylamins, sowie
(c) 0 bis 30 mol % weiterer radikalisch polymerisierbarer Monomerer
handelt und die Summe aus (a), (b) und (c) 100 mol % ergibt.

8. Verfahren gemäß Anspruch 7, **dadurch gekennzeichnet, dass** die antimikrobielle Zusammensetzung zusätzlich mindestens ein Bindemittel umfasst.

9. Verfahren gemäß Anspruch 7 oder 8, **dadurch gekennzeichnet, dass** die antimikrobielle Zusammensetzung zusätzlich einen Vernetzer oder ein Vernetzersystem umfasst.

## Claims

1. A method of killing microorganisms in aqueous industrial systems or products for industrial applications based on water by adding a biocidal additive to the system or product, wherein the biocide is from 0.001 to 5% by weight of at least one water-soluble or water-dispersible polymer comprising - based in each case on the total amount of all monomer units present in the polymer
(a) from 30 to 98 mol% of styrenesulfonic acid,
(b) from 2 to 40 mol% of an N-vinyllactam and/or N-vinylamine, and
(c) from 0 to 30 mol% of further free-radically polymerizable monomers
and the sum of (a), (b), and (c) makes 100 mol%.

2. The method according to claim 1, wherein all or some of the sulfonic acid groups are in salt form.

3. The method according to claim 1 or 2, wherein the industrial products based on water are aqueous dispersions.

4. The method according to claim 3, wherein the dispersion is electrostatically or ionically stabilized.

5. The method according to claim 3, wherein the dispersion is spray dried.

6. The method according to claim 1 or 2, wherein the aqueous industrial systems are refrigeration or heat exchanger circuits.

7. A method of protecting articles by applying an antimicrobial composition at least comprising water or a predominantly hydrous solvent mixture and a biocidal additive to the article by means of an appropriate technique and removing water or the predominantly hydrous solvent mixture, wherein the biocide is from 0.001 to 5% by weight of at least one water-soluble or water-dispersible polymer comprising - based in each case on the total amount of all monomer units present in the polymer
(a) from 30 to 98 mol% of styrenesulfonic acid,
(b) from 2 to 40 mol% of an N-vinyllactam and/or N-vinylamine, and
(c) from 0 to 30 mol% of further free-radically polymerizable monomers
and the sum of (a), (b), and (c) makes 100 mol%.

8. The method according to claim 7, wherein the antimicrobial composition further comprises at least one binder.

9. The method according to claim 7 or 8, wherein the antimicrobial composition further comprises a crosslinker or a system of crosslinkers.

## Revendications

1. Procédé de destruction de microorganismes dans des systèmes techniques aqueux ou des produits pour des applications techniques à base d'eau, selon lequel un additif biocide est ajouté au système ou au produit, **caractérisé en ce que** le biocide est 0,001 à 5 % en poids d'au moins un polymère soluble dans l'eau ou dispersible dans l'eau contenant, à chaque fois par rapport à la quantité totale de toutes les unités monomères présentes dans le polymère,
(a) 30 à 98 % en moles d'acide styrènesulfonique,
(b) 2 à 40 % en moles d'un N-vinyllactame et/ou d'une N-vinylamine, ainsi que
(c) 0 à 30 % en moles de monomères polymérisables radicalairement supplémentaires,
la somme de (a), (b) et (c) étant de 100 % en moles.

2. Procédé selon la revendication 1, **caractérisé en ce que** les groupes acide sulfonique se présentent en totalité ou en partie sous la forme d'un sel.

3. Procédé selon la revendication 1 ou 2, **caractérisé en ce que** les produits techniques à base d'eau sont des dispersions aqueuses.

4. Procédé selon la revendication 3, **caractérisé en ce que** la dispersion est stabilisée électrostatiquement ou ioniquement.

5. Procédé selon la revendication 3, **caractérisé en ce que** la dispersion est séchée par pulvérisation.

6. Procédé selon la revendication 1 ou 2, **caractérisé en ce que** les systèmes techniques aqueux sont des circuits de refroidissement ou d'échange de chaleur.

7. Procédé de protection d'articles, selon lequel une composition à action antimicrobienne, qui comprend au moins de l'eau ou un mélange de solvants contenant principalement de l'eau et un additif biocide, est appliquée par un procédé approprié sur l'article et l'eau ou le mélange de solvants contenant principalement de l'eau est éliminé, **caractérisé en ce que** le biocide est 0,001 à 5 % en poids d'au moins un polymère soluble dans l'eau ou dispersible dans l'eau contenant, à chaque fois par rapport à la quantité totale de toutes les unités monomères présentes dans le polymère,
(a) 30 à 98 % en moles d'acide styrènesulfonique,
(b) 2 à 40 % en moles d'un N-vinyllactame et/ou d'une N-vinylamine, ainsi que
(c) 0 à 30 % en moles de monomères polymérisables radicalairement supplémentaires,
la somme de (a), (b) et (c) étant de 100 % en moles.

8. Procédé selon la revendication 7, **caractérisé en ce que** la composition antimicrobienne comprend également au moins un liant.

9. Procédé selon la revendication 7 ou 8, **caractérisé en ce que** la composition antimicrobienne comprend
également un agent de réticulation ou un système de réticulation.
